# FASCICULE DE BREVET EUROPEEN

(11) **EP 4 356 098 B1**
(45) Date de publication et mention de la délivrance du brevet: **17.12.2025**
(21) Numéro de dépôt: 22741329.1
(22) Date de dépôt: 13.06.2022
(51) Int. Cl.: G01N 7/04, G01N 7/22, G01N 33/10

(54) **APPAREIL POUR LA DETERMINATION DE LA QUANTITE DE CO2 ABSORBEE PAR UN ECHANTILLON DE MATIERE AU COURS DU TEMPS**
VORRICHTUNG ZUR BESTIMMUNG DER VON EINER MATERIALPROBE IM LAUFE DER ZEIT ABSORBIERTEN CO2-MENGE
DEVICE FOR DETERMINING THE QUANTITY OF CO2 ABSORBED BY A SAMPLE OF MATERIAL OVER TIME

(30) Priorité: 15.06.2021 FR 2106288
(43) Date de publication de la demande: 24.04.2024
(73) Titulaire: Lesaffre et Compagnie, 75001 Paris (FR)
(72) Inventeur: BARTOLUCCI, Jean-Charles, 59560 Hellemmes-Lille (FR); KAPRAL, Florence, 59560 Comines (FR)
(74) Mandataire: Plasseraud IP
(86) Numéro de dépôt international: PCT/FR2022/051128
(87) Numéro de publication internationale: WO 2022/263761

(56) Documents cités:
- FR-A1- 2 528 175
- GB-A- 495 849
- US-A- 2 038 044

## Description

### Domaine technique

Un aspect de la présente invention est relative à un contenant pour la détermination au cours du temps de la quantité de dioxyde de carbone (CO₂) absorbée et/ou expulsée par un échantillon de matière, et notamment de matière organique contenant un agent levant, comme par exemple de la levure et/ou du levain et/ou de la poudre levante, comme par exemple de la pâte boulangère contenant de la levure boulangère.

L'invention concerne un appareil pour la détermination au cours du temps de la quantité de CO₂ absorbée par un échantillon de matière comprenant un tel contenant.

L'invention concerne encore un procédé de détermination au cours du temps de la quantité de CO₂ absorbée et/ou expulsée par un échantillon de matière.

### Technique antérieure

La principale fonctionnalité d'un agent levant, comme par exemple de la levure et/ou du levain et/ou de la poudre levante, est de faire lever la pâte boulangère. Pour ce faire, il produit du CO₂ qui sature rapidement la phase liquide de la matrice pâteuse et passe en phase vapeur au sein des nombreux nucléi présents dans la pâte en fin de pétrissage, produisant ainsi leur expansion, et qui donneront lieu dans le produit final aux nombreuses alvéoles de la mie de pain. Cette expansion n'est possible que par la capacité de rétention du gaz de la pâte boulangère, en particulier à base de farine de blé.

L'expansion de la pâte est facilement observable. En plaçant une masse déterminée de pâte dans une éprouvette et en mesurant régulièrement la hauteur de la pâte dans l'éprouvette, on peut globalement évaluer cette expansion, au niveau macroscopique. Cette mesure n'est pas très précise et du fait de son caractère macroscopique, intègre d'autres phénomènes tels que la rhéologie de la pâte. Par exemple, pour un même volume de pâte, on peut avoir des hauteurs de pâte différentes (pousse « plate » ou « ronde ») et des poches de gaz peuvent éventuellement se loger entre la pâte et l'éprouvette ce qui peut fausser les mesures.

Il existe depuis longtemps des appareils qui permettent de mesurer la production globale de CO₂ par la levure dans la pâte boulangère. Leur principe est le suivant : on place un morceau de pâte de masse connue dans un pot hermétique ; puis lorsque la levure produit du CO₂, cela engendre une surpression dans le pot, d'une part parce que le volume de pâte augmente, comprimant ainsi le ciel gazeux dans le pot, et d'autre part parce que du CO₂ est expulsé par la pâte boulangère dans le ciel gazeux du pot, augmentant ainsi le nombre de molécules de CO₂ présentes dans celui-ci. Cette surpression peut être quantifiée par le déplacement d'un liquide, ce qui correspond à une mesure directe de volume, et/ou par l'emploi d'un capteur de pression, comme dans certains appareils de mesure connus, comme par exemple l'appareil de mesure commercialisé sous le nom de RISOGRAPH ^{®} ou de RHÉOFERMENTOMÈTRE ^{®}. Le principal intérêt de ces appareils est de donner une vision cinétique du phénomène de production de CO₂ par une pâte boulangère au cours du temps.

Cependant, la mesure de la production totale de CO₂ par une pâte boulangère ne distingue pas la partie retenue par la pâte (donnant lieu à l'expansion de la pâte) de celle perdue dans l'atmosphère. Elle renseigne donc parfaitement sur l'activité fermentaire de la levure, mais pas sur la part de CO₂ retenue dans la pâte. Or, de nombreux facteurs liés à la recette de panification (à commencer par la qualité de la farine) comme au process (exemple : le cru surgelé), modulent fortement cette rétention/expulsion du gaz. Avoir une vue précise de la cinétique de la rétention/expulsion gazeuse, en dehors des aspects rhéologiques, est donc indispensable pour pouvoir être en mesure de découpler les effets liés à l'activité fermentaire de la levure des propriétés de la matrice pâteuse.

Le RHÉOFERMENTOMÈTRE ^{®}, cité plus haut, a tenté de proposer une solution à ce problème. Une telle solution consiste à mesurer simultanément la variation de pression dans le ciel gazeux du pot contenant la pâte boulangère via un canal direct, pour obtenir la production totale de CO₂ par la pâte boulangère, et via un canal passant par un piège à CO₂, comprenant par exemple de la chaux sodée et ainsi ne mesurer que la surpression due à la seule expansion de la pâte boulangère (induisant de facto la compression du ciel gazeux du pot). Dans un tel appareil, le piège à CO₂ est connecté au pot contenant la pâte boulangère via une conduite, et se trouve donc écarté dudit pot et donc du ciel gazeux du pot contenant l'échantillon de pâte boulangère.

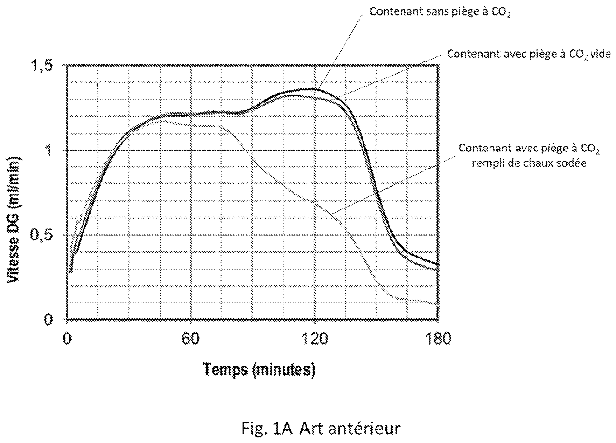

Les courbes représentées sur le graphique de la figure 1A montrent une estimation de l'évolution au cours du temps de la vitesse de dégagement de CO₂ d'un échantillon de pâte boulangère dans ce type de dispositif.

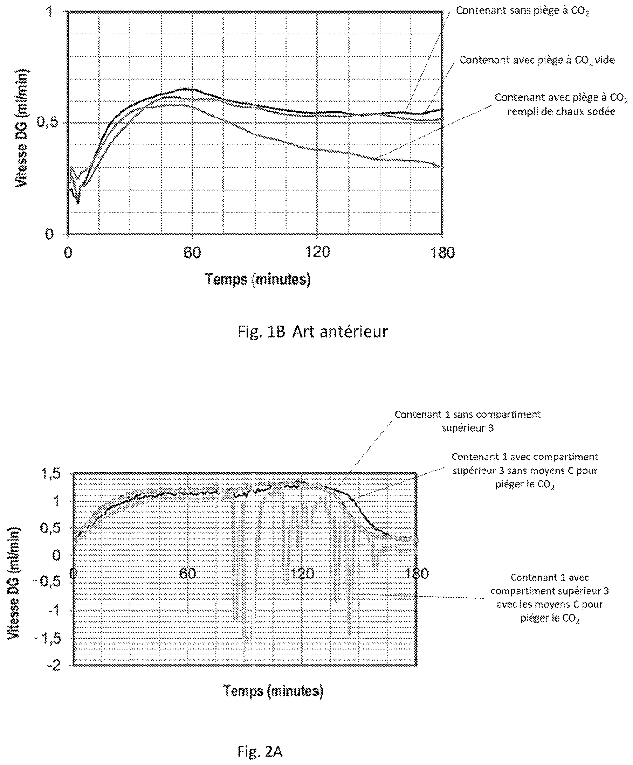

Cette solution est néanmoins peu satisfaisante pour rendre compte de l'évolution au cours du temps de la rétention/expulsion de CO₂ par une pâte boulangère. En effet, et selon les constatations de l'inventeur, et comme visible sur les courbes du graphique de la figure 1B : si l'on remplace la pâte boulangère à base de farine par un système témoin contenant de la levure mais incapable de retenir le CO₂, comme par exemple un bécher contenant de l'eau, du sucre et de la levure, la courbe représentative de la rétention du CO₂ par ledit système témoin, obtenue par les mesures de pression via le canal avec piège à CO₂, qui devrait stagner autour d'une valeur nulle (puisque l'eau ne gonfle pas lorsque le sucre est transformé en CO2 par la levure), peut en réalité sensiblement se superposer à la courbe représentative de l'augmentation de la pression totale dans le pot obtenue par les mesures de pression via le canal direct, en particulier aux temps courts. Tout se passe comme si le système (le bécher contenant une solution sucrée levurée) retenait le CO₂ pendant plus d'une heure, ce qui n'est pas exact. Cet appareil ne remplit donc pas la mission de renseigner pleinement sur l'apparition de fuites de CO₂ dans une pâte boulangère, du moins d'une manière absolue, et ne peut donc être utilisé pour répondre avec précision à l'objectif de suivre la cinétique de rétention/expulsion de CO₂ dans une pâte boulangère, et plus généralement dans un échantillon de matière, et notamment de matière organique contenant de la levure, au cours du temps de fermentation.

On connaît encore du document FR2528175, publié en 1983 un procédé qui permet de mesurer la qualité rhéologique de la pâte fermentée et de prévoir le comportement de cette pâte lors du choc thermique de la cuisson. Le procédé permet également d'évaluer le comportement de la pâte lors de la poussée gazeuse, ainsi que les effets des additifs et améliorant les farines.

Le dispositif pour la mise en œuvre du procédé comprend :
- une cuve thermostatée destinée à contenir la pâte ainsi qu'une masse pesante et des moyens pour mesurer le déplacement et enregistrer le déplacement de la masse en appui sur la pâte lors de sa pousse,
- des moyens pour mesurer et enregistrer en même temps la vitesse de dégagement du gaz formé lors de la fermentation de la pâte et libéré de la pâte, ainsi que la vitesse du gaz débarrassé du gaz carbonique.

Ces moyens de mesure comportent un ajutage sur le couvercle de la cuve qui est relié par une conduite à l'entrée d'un distributeur à quatre voies, commandé par un moteur. L'une des voies de sortie du distributeur est connectée par une première conduite à un capteur de pression qui commande l'enregistreur. Une autre voie est reliée au capteur, par une conduite sur laquelle est interposé un piège à CO₂ comprenant un vase d'absorption contenant de la potasse. Lorsque le distributeur tourne, la conduite de l'ajutage est successivement reliée à la conduite en lien direct au capteur de pression, puis à l'atmosphère, puis à la conduite en lien indirect au capteur de pression par le piège à CO2, puis de nouveau en lien direct avec le capteur, et ainsi de suite.

Un tel dispositif permet de distinguer le moment où les protéines de la pâte ne peuvent plus supporter les contraintes dues à la fermentation et que la pâte retombe en libérant le CO₂, à savoir lorsque deux courbes se séparent, une première courbe représentant l'évolution de la pression lorsque le distributeur est connecté directement au capteur de pression, la deuxième courbe représentant l'évolution de le pression lorsque le distributeur est connecté au capteur de pression, mais seulement par la piège à CO₂, le rapport des ordonnées indiquant alors la teneur en CO₂ repoussé (expulsé) par la pâte.

Le FR2528175 repose, à l'instar du RHÉOFERMENTOMÈTRE ^{®}, sur un piège à CO₂ situé à distance de l'enceinte contenant la pâte, et sur l'hypothèse que le gaz qui se dégage de la pâte a été absorbé par le piège à CO₂, ce qui est en réalité, peu fiable et comme exposé sur les courbes de la figure 1B réalisées par les inventeurs.

Il existe par ailleurs une autre approche technique via l'emploi d'un ou plusieurs capteurs de CO₂ dans l'atmosphère (consistant par exemple en une ou plusieurs sondes infrarouges) permettant de déduire la cinétique de rétention/expulsion du CO₂ d'un échantillon de matière, et notamment de pâte boulangère contenant un agent levant, comme par exemple de la levure et/ou du levain et/ou de la poudre levante. Leur exploitation est complexe techniquement, puisqu'il faut compenser de manière dynamique la modification du volume du ciel gazeux dans le pot contenant la pâte boulangère du fait de l'expansion de l'échantillon de matière. La comparaison en parallèle de la production totale de CO₂ dans le pot est délicate, les deux informations étant de nature très différentes (mesure de pression pour la détermination de la production totale de CO₂ par la pâte boulangère dans le pot et mesure de la concentration en CO₂ dans le ciel gazeux de volume variable pour la détermination de la quantité de CO₂ retenue/expulsée par la pâte boulangère dans le pot). Certains appareils connus emploient une telle technologie comme par exemple l'appareil commercialisé sous le nom de BLUESENS^{®}.

Il existe encore du document GB 495 849, publié en 1938, un appareil pour la mesure de l'évolution de la quantité de gaz libéré dans une fermentation, en particulier une fermentation de pâte boulangère, l'appareil comportant deux conteneurs étanches au gaz, l'un des conteneurs permettant la mesure de la totalité du gaz produit par une première fraction de pâte, et l'autre conteneur la mesure du gaz qui se forme lors de la fermentation, mais qui reste retenu par la pâte. Chaque conteneur est totalement étanche au gaz et comprend une partie inférieure, fixe, et une partie supérieure sous forme d'une cloche. La cloche coulisse verticalement de manière étanche par rapport à la partie inférieure du conteneur, en fonction de l'évolution de la pression interne. L'étanchéité entre la partie fixe et la partie mobile du conteneur est obtenue par immersion du conteneur dans une bain d'huile mis en circulation par une pompe et thermorégulé. L'évolution de la quantité de gaz généré dans chaque conteneur est mesurée, mécaniquement, par un système de poulie et contrepoids permettant la lecture de l'expansion du volume du gaz dans la cloche.

### Problème technique

L'objectif de l'invention est donc de pallier les inconvénients des techniques de l'art antérieur de détermination au cours du temps de la quantité de CO₂ retenue/expulsée par un échantillon de matière, et notamment de matière organique contenant un agent levant, comme par exemple de la levure et/ou du levain et/ou de la poudre levante, en permettant de déterminer de manière plus fiable et plus simple au cours du temps la quantité de CO₂ retenue/expulsée par un échantillon de matière, et notamment de matière organique contenant un agent levant, comme par exemple de la levure et/ou du levain et/ou encore de la poudre levante.

Un autre but de la présente invention est de permettre de déterminer au cours du temps la quantité de CO₂ retenue/expulsée par un échantillon de matière, et notamment de matière organique contenant un agent levant, comme par exemple de la levure et/ou du levain et/ou de la poudre levante, pour un coût de revient réduit.

### Exposé de l'invention

Il est proposé un appareil pour la détermination de la quantité de CO₂ absorbée par un échantillon de matière organique contenant de la levure et/ou du levain et/ou de la poudre levante, en particulier de pâte boulangère, au cours du temps, comprenant :
- un moyen de mesure de pression , et
- au moins un contenant comprenant :
   - - un compartiment inférieur prévu pour recevoir un échantillon de matière,
   - - un compartiment supérieur recevant des moyens pour piéger le CO₂, positionné dans le prolongement et communiquant avec le compartiment inférieur, et présentant une ouverture d'échappement permettant au gaz de s'échapper du compartiment supérieur après son passage par lesdits moyens pour piéger le CO₂,
   - - un moyen de séparation, disposé entre le compartiment inférieur et le compartiment supérieur, configuré pour autoriser le passage de gaz depuis le compartiment inférieur vers le compartiment supérieur,
dans lequel le moyen de mesure de la pression est relié à l'ouverture d'échappement du compartiment supérieur du contenant par une conduite souple de sorte à pouvoir déterminer l'évolution au cours du temps de la pression dans le compartiment inférieur recevant l'échantillon de matière, après passage des gaz issus du compartiment inférieur dans le compartiment supérieur du contenant.

Selon des caractéristiques optionnelles de l'invention, prises seules ou en combinaison :
- le compartiment inférieur est au moins partiellement, de préférence intégralement, dans un matériau transparent, par exemple en verre ou encore en matériau thermoplastique, de sorte à permettre à un observateur de visualiser le contenu du compartiment inférieur, et en particulier de suivre la pousse de la pâte lors de la fermentation,
- le compartiment inférieur, le compartiment supérieur et le moyen de séparation forment un ensemble autoportant ;
- le moyen de séparation comporte un tamis dans lequel sont ménagées une pluralité d'ouvertures configurées de sorte à autoriser le passage de gaz depuis le compartiment inférieur vers le compartiment supérieur ;
- les moyens pour piéger le CO₂ comportent des granulés de chaux sodée ;
- les granulés de chaux sodée sont disposés directement sur le tamis, les ouvertures du tamis étant configurées de sorte à empêcher le passage des granulés de chaux sodée du compartiment supérieur au compartiment inférieur ;
- le contenant comporte une ouverture supérieure et le compartiment supérieur comporte une ouverture inférieure, l'ouverture supérieure du contenant étant alignée avec l'ouverture inférieure du compartiment supérieur, et dans lequel le moyen de séparation est également aligné avec l'ouverture supérieure du contenant et avec l'ouverture inférieure du compartiment supérieur ;
- un bouchon amovible est positionné dans l'ouverture supérieure du contenant, de sorte à rendre étanche ledit contenant ;
- le bouchon comporte un perçage traversant positionné dans le prolongement de l'ouverture d'échappement du compartiment supérieur, de sorte à permettre au gaz du compartiment supérieur de s'en échapper via ladite ouverture d'échappement ;
- le moyen de séparation est disposé au niveau de l'ouverture inférieure du compartiment supérieur ;
- le moyen de séparation est solidarisé, et notamment de manière amovible, au compartiment supérieur, éventuellement par l'intermédiaire de moyens de solidarisation ;
- le compartiment inférieur et le compartiment supérieur sont fixés de manière amovible ;
- le contenant comporte une paroi périphérique de forme sensiblement cylindrique, et le compartiment supérieur comporte également une paroi périphérique de forme sensiblement cylindrique, d'axe confondu avec l'axe de la paroi périphérique du contenant ;
- le contenant comprend en outre un moyen de raccordement, notamment amovible, configuré pour permettre le raccordement du compartiment supérieur à la conduite souple, ledit moyen de raccordement étant positionné au niveau de l'ouverture d'échappement du compartiment supérieur, en s'étendant au travers de ladite ouverture d'échappement ;
- le moyen de raccordement est maintenu en position par rapport au compartiment supérieur par ladite ouverture d'échappement du compartiment supérieur, ledit moyen de raccordement s'étendant au travers du perçage traversant du bouchon et étant maintenu en position par rapport au bouchon par ledit perçage traversant ;
- le compartiment supérieur comporte une paroi supérieure, l'ouverture d'échappement étant ménagée dans ladite paroi supérieure.

L'invention concerne encore un procédé de détermination au cours du temps de la quantité de CO₂ absorbée par un échantillon de matière, comprenant de la matière organique contenant de la levure et/ou du levain et/ou de la poudre levante, en particulier de pâte boulangère, comprenant :
/a/ fourniture d'un appareil selon l'une des modes de réalisation de l'invention ;
/b/ mise en place de l'échantillon de matière dans le compartiment inférieur du contenant,
/c/ mesure de l'évolution de la pression au cours du temps dans le compartiment inférieur recevant l'échantillon de matière, après passage des gaz issus du compartiment inférieur dans le compartiment supérieur du contenant.

Lors de la mise en œuvre un tel procédé, la variation de pression est due à la fermentation génératrice de CO₂, qui est retenu par l'échantillon (à savoir le CO₂ absorbé), et non au CO₂ expulsé par l'échantillon puisque le CO₂ expulsé est éliminé par les moyens pour piéger le CO₂ dans le compartiment supérieur.

La réaction de fermentation ne générant que du gaz CO₂, on peut déterminer par calcul une quantité de CO₂ absorbé par la connaissance du volume interne du contenant, auquel on additionne, le volume interne de la conduite souple reliant l'ouverture d'échappement au moyen de mesure de pression.

La présente divulgation concerne encore un procédé de détermination au cours du temps de la quantité de CO₂ expulsé par un échantillon de matière organique contenant de la levure et/ou du levain et/ou de la poudre levante, en particulier de pâte boulangère au cours d'une réaction de fermentation génératrice de CO₂, comprenant simultanément, une première mesure et une deuxième mesure sur une première fraction d'échantillon et une deuxième fraction d'échantillon, de même volume, et dans lequel ladite première mesure est configurée pour mesurer l'évolution de pression due à la quantité de gaz absorbé uniquement, et la deuxième mesure est configurée pour mesurer l'évolution de pression due à la quantité de gaz absorbé et à la quantité de gaz expulsé et dans lequel la première mesure comprend :
/a1/ fourniture d'un premier appareil selon l'une des revendications 1 à 15 comprenant les moyens pour piéger le CO₂ reçus dans le compartiment supérieur du contenant ;
/b1/ mise en place de la première fraction de échantillon de matière (M) dans le compartiment inférieur du contenant ,
/c1/ mesure de l'évolution de la pression au cours du temps dans le compartiment inférieur recevant la première fraction de l'échantillon de matière, après passage des gaz issus du compartiment inférieur dans le compartiment supérieur du contenant, le CO₂ expulsé par l'échantillon éliminé par les moyens pour piéger le CO₂
et dans lequel la deuxième mesure comprend :
   /a2/ fourniture d'un deuxième appareil selon l'une des revendications 1 à 15 dépourvu de moyens pour piéger le CO₂ reçus dans le compartiment supérieur (3) du contenant ;
   /b2/ mise en place de la deuxième fraction de échantillon de matière dans le compartiment inférieur du contenant,
   /c2/ mesure de l'évolution de la pression au cours du temps dans le compartiment inférieur recevant la deuxième fraction de échantillon de matière, après passage des gaz issus du compartiment inférieur dans le compartiment supérieur (3) du contenant, dépourvu des moyens pour piéger le CO₂
   et dans lequel on obtient la quantité de CO₂ expulsé par l'échantillon à partir de la différence entre la deuxième mesure et la première mesure.

### Brève description des dessins

D'autres caractéristiques, détails et avantages de l'invention apparaîtront à la lecture de la description détaillée ci-après, et à l'analyse des dessins annexés, sur lesquels :
**Fig. 1A**
   [Fig. 1A] montre un graphique représentant l'évolution au cours du temps de la vitesse du dégagement de CO₂ par un échantillon de pâte boulangère établi avec un appareil de l'art antérieur.
**Fig. 1B**
   [Fig. 1B] montre un graphique représentant l'évolution au cours du temps de la vitesse du dégagement de CO₂ par un échantillon d'eau + levure + sucre établi avec un appareil de l'art antérieur.
**Fig. 2A**
   [Fig. 2A] montre un graphique représentant l'évolution au cours du temps de la vitesse du dégagement de CO₂ par un échantillon de pâte boulangère établi avec un appareil selon l'invention.
**Fig. 2B**

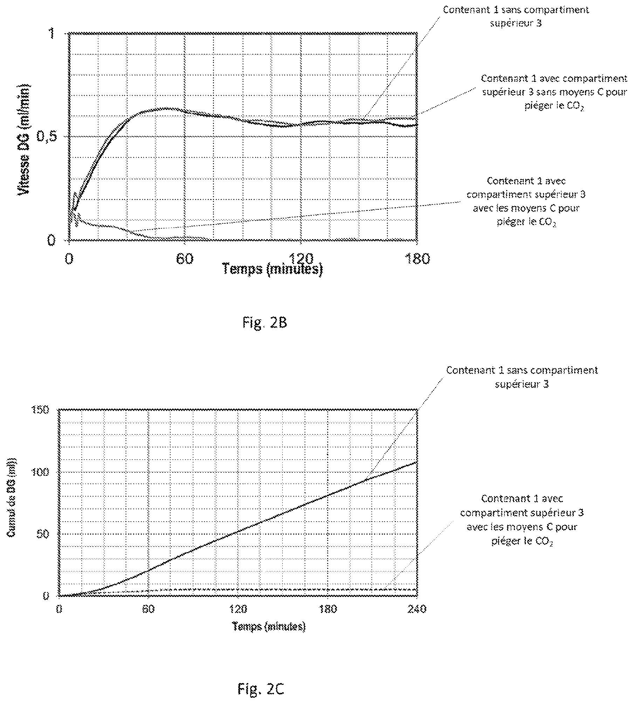

   [Fig. 2B] montre un graphique représentant l'évolution au cours du temps de la vitesse du dégagement de CO₂ par un échantillon d'eau + levure +sucre établi avec un appareil selon l'invention.
**Fig. 2C**
   [Fig. 2C] montre un graphique représentant l'évolution au cours du temps de la quantité cumulée de CO₂ dégagée par un échantillon d'eau + levure + sucre établi avec un appareil selon l'invention.
**Fig. 2D**

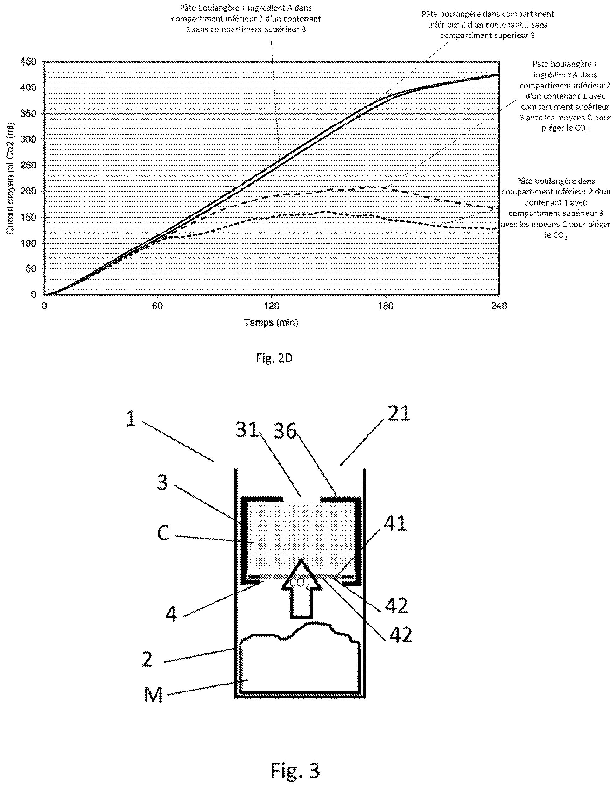

   [Fig. 2D] montre un graphique représentant l'évolution au cours du temps de la quantité cumulée de CO₂ dégagée par un échantillon de pâte boulangère avec ou sans un ingrédient A.
**Fig. 3**
   [Fig. 3] montre une vue schématique d'un contenant selon un mode de réalisation d'un aspect de l'invention.
**Fig. 4**

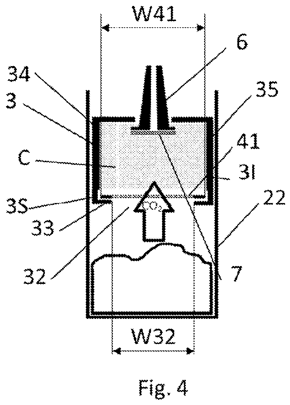

   [Fig. 4] montre une vue schématique d'un contenant selon un mode de réalisation d'un aspect de l'invention.
**Fig. 5**

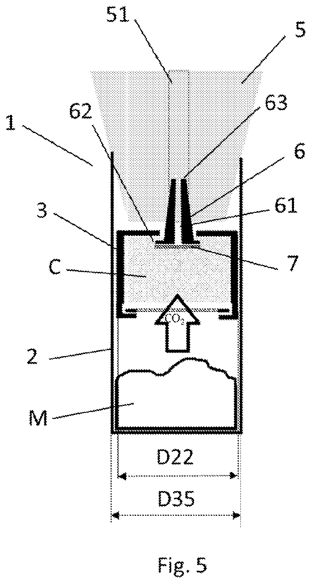

   [Fig. 5] montre une vue schématique d'un contenant selon un mode de réalisation d'un aspect de l'invention.
**Fig. 6**

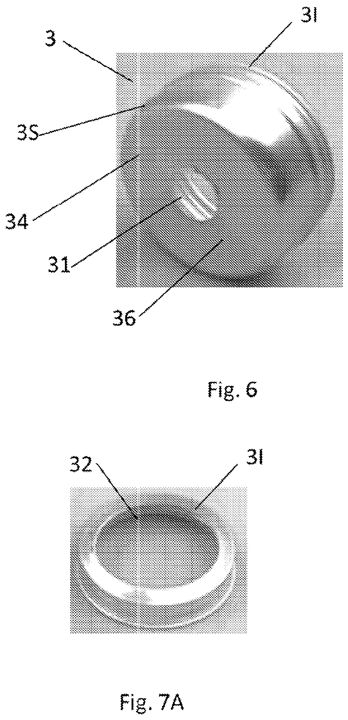

   [Fig. 6] montre une vue en perspective du compartiment supérieur d'un contenant selon un mode de réalisation d'un aspect de l'invention.
**Fig. 7A**
   [Fig. 7A] montre une vue en perspective de la partie inférieure du compartiment supérieur d'un contenant selon un mode de réalisation d'un aspect de l'invention
**Fig. 7B**

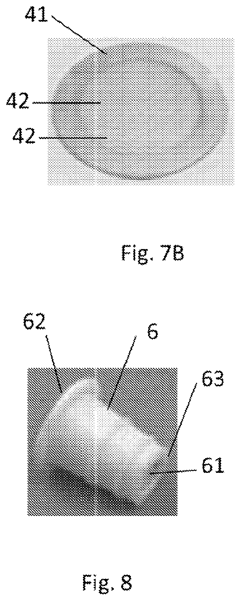

   [Fig. 7B] montre une vue en perspective du tamis d'un contenant selon un mode de réalisation d'un aspect de l'invention.
**Fig. 8**
   [Fig. 8] montre une vue en perspective du moyen de raccordement d'un contenant selon un mode de réalisation d'un aspect de l'invention.
**Fig. 9A**

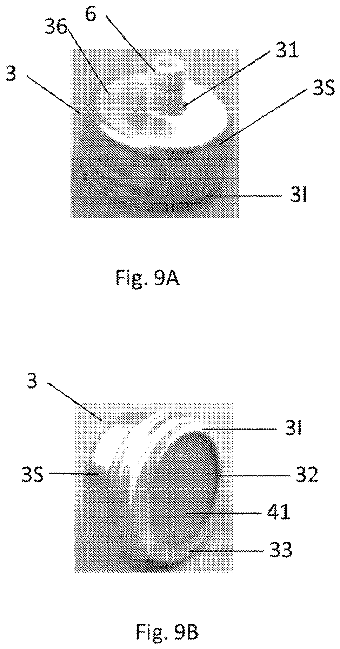

   [Fig. 9A] montre une vue en perspective du compartiment supérieur d'un contenant selon un mode de réalisation d'un aspect de l'invention.
**Fig. 9B**
   [Fig. 9B] montre une vue en perspective du compartiment supérieur d'un contenant selon un mode de réalisation d'un aspect de l'invention.
**Fig. 10**

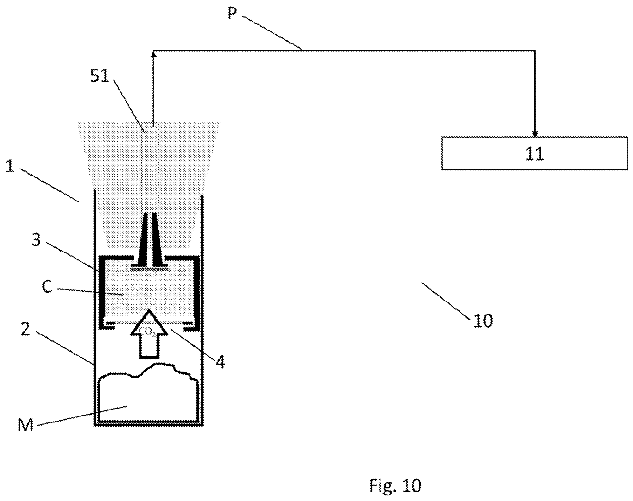

   [Fig. 10] montre une vue en perspective d'un appareil selon un mode de réalisation de l'invention.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Dans l'ensemble de la présente demande, supérieur/inférieur et latéral, en ce qui concerne la position de certains éléments du contenant s'entendent dans la position normale d'utilisation du contenant selon l'invention, avec le compartiment supérieur positionné au-dessus du compartiment inférieur selon une direction sensiblement verticale de l'espace.

Un aspect de l'invention concerne un contenant 1 pour la détermination de la quantité de CO₂ absorbée par un échantillon de matière M comprenant:
- un compartiment inférieur 2 prévu pour recevoir un échantillon de matière M, et notamment de la matière organique contenant un agent levant, comme par exemple de la levure et/ou du levain et/ou de la poudre levante, en particulier de la pâte boulangère (i.e. un mélange de farine, eau, sel, levure, etc.),
- un compartiment supérieur 3 recevant des moyens C pour piéger le CO₂, positionné dans le prolongement et communiquant avec le compartiment inférieur 2, et présentant une ouverture d'échappement 31 permettant au gaz de s'échapper du compartiment supérieur 3 après son passage par lesdits moyens C pour piéger le CO₂,
- un moyen de séparation 4, disposé entre le compartiment inférieur 2 et le compartiment supérieur 3, configuré pour autoriser le passage de gaz depuis le compartiment inférieur 2 vers le compartiment supérieur 3.

Un tel contenant 1 permet avantageusement de positionner les moyens C pour piéger le CO₂ au plus proche de l'échantillon de matière M. Ainsi, l'ensemble du CO₂ expulsé par l'échantillon de matière M au cours du temps est piégé par les moyens C pour piéger le CO₂.

De ce fait, en effectuant par exemple une mesure de pression en sortie du compartiment supérieur 3, en aval de l'ouverture d'échappement 31, la pression mesurée reflète donc uniquement la capacité de rétention de CO₂ par l'échantillon de matière M, et sa variation reflète de manière avantageuse la cinétique de rétention de CO₂ par l'échantillon de matière M, et comme visible plus particulièrement sur les courbes des graphiques des figures 2A (sur pâte boulangère), 2B et 2C (sur système liquide eau + levure + sucre).

Par ailleurs, et selon les constatations de l'inventeur, si l'on place dans le compartiment inférieur 2 du contenant 1 un échantillon d'un système témoin contenant de la levure mais incapable de retenir le CO₂, tel que décrit plus haut, comme par exemple un bécher contenant de l'eau, du sucre et de la levure, la courbe représentative de la rétention du CO₂ par ledit système témoin, obtenue par les mesures de pression via l'ouverture d'échappement 31 du compartiment supérieur 3 du contenant 1, stagne autour d'une valeur sensiblement nulle, comme visible sur les courbes des graphiques des figures 2B et 2C, ce qui est conforme à ce qui se passe dans ledit compartiment inférieur 2, puisque l'eau ne retient pas le CO₂, et contrairement aux mesures effectuées avec un appareil et un contenant de l'art antérieur, comme expliqué ci-dessus, et comme visible sur les courbes du graphique de la figure 1B.

Ainsi, l'ensemble du CO₂ expulsé par un échantillon de matière M, et notamment de pâte boulangère, disposé dans le compartiment inférieur 2, est donc piégé par les moyens C pour piéger le CO₂, disposés dans le compartiment supérieur 3.

Le contenant 1 selon un aspect de l'invention s'avère donc particulièrement avantageux pour déterminer l'évolution au cours du temps de la quantité de CO₂ retenue par un échantillon de matière M, et notamment de matière organique contenant un agent levant, comme par exemple de la levure et/ou du levain et/ou de la poudre levante, en particulier de pâte boulangère, de manière fiable, et comme visible par exemple sur les courbes des graphiques des figures 2A à 2C.

Ainsi, il devient possible de déterminer de manière particulièrement fiable, l'influence de différents paramètres sur l'évolution au cours du temps de la quantité de CO₂ retenue par un échantillon de matière M, et notamment de matière organique contenant un agent levant, comme par exemple de la levure et/ou du levain et/ou de la poudre levante, en particulier de pâte boulangère. Notamment, comme visible sur le graphique de la figure 2D, les inventeurs ont pu déterminer, grâce au contenant 1 selon l'invention, que l'ajout d'un ingrédient A dans une composition de pâte boulangère, n'avait pas d'influence sur la production totale de CO₂ par la pâte boulangère (les deux courbes en traits pleins sont sensiblement superposées), mais avait une influence sur la quantité de CO₂ retenue par ladite pâte boulangère au cours du temps (l'échantillon de pâte boulangère contenant l'ingrédient A retient plus de CO₂ au cours du temps que l'échantillon de pâte boulangère qui en est dépourvu, comme le montrent les deux courbes en pointillés). Cela n'aurait pas été possible avec les techniques et appareils de l'art antérieur.

De plus, un tel contenant 1 présente une conception particulièrement simple ce qui lui permet de présenter un coût de revient réduit.

Le compartiment inférieur 2 peut avantageusement être réalisé, au moins partiellement, de préférence intégralement, dans un matériau transparent, par exemple en verre ou encore en matériau thermoplastique, de sorte à permettre à un observateur de visualiser le contenu du compartiment inférieur 2.

Le compartiment supérieur 3 peut avantageusement être réalisé, au moins partiellement, de préférence intégralement, dans un matériau rigide, comme par exemple un matériau plastique ou encore un matériau métallique, et notamment en aluminium.

Selon un mode de réalisation, le compartiment inférieur 2, le compartiment supérieur 3 et le moyen de séparation 4 forment un ensemble autoportant.

Cette disposition avantageuse facilite la manutention, l'utilisation et la fabrication du contenant 1 selon l'invention.

Selon un mode de réalisation, et comme visible plus particulièrement sur les figures 7B et 9B, le moyen de séparation 4 comporte un tamis 41 dans lequel sont ménagées une pluralité d'ouvertures 42 configurées de sorte à autoriser le passage de gaz depuis le compartiment inférieur 3 vers le compartiment supérieur 2.

Le moyen de séparation 4 présente ainsi une conception simple et un coût de revient réduit.

Le tamis 41 peut par exemple être réalisé en métal, et notamment en acier inoxydable.

Avantageusement, les ouvertures 42 peuvent présenter une largeur comprise entre 150 µm et 350 µm, de préférence entre 200 µm et 300 µm.

Afin de faciliter l'accès auxdits moyens C pour piéger le CO₂, ainsi qu'éventuellement auxdits moyens de séparation 4, le compartiment supérieur 3 peut être avantageusement réalisé en deux parties amovibles : une partie supérieure 3S et une partie inférieure 3I. Un filetage peut avantageusement être ménagé sur le pourtour de la partie supérieure 3S et sur le pourtour de la partie inférieure 31, de sorte à pouvoir assembler/désassembler la partie inférieure 3I et la partie supérieure 3S du compartiment supérieur 3 par vissage/dévissage.

Selon un mode de réalisation, et comme visible plus particulièrement sur les figures 3 à 5, les moyens C pour piéger le CO₂ comportent des granulés C de chaux sodée.

Les granulés de chaux sodée sont des éléments bien connus qui permettent de piéger du CO₂. Les composants principaux de la chaux sodée sont généralement l'hydroxyde de calcium : Ca(OH)₂ et l'hydroxyde de sodium : NaOH.

Les granulés de chaux sodés sont particulièrement efficaces pour piéger le CO₂, en ce qu'ils réagissent avec celui-ci pour former de l'eau, selon les réactions chimiques suivantes :

H₂O + CO₂ = H⁺ + HCO₃

NaOH + H₂CO₃ = NaHCO₃ + H₂O

2NaHCO₃ + Ca(OH)₂₊ = 2NaOH + CaCO₃ + H₂O

L'emploi de granulés de chaux sodée comme moyens C pour piéger le CO₂ présente également l'avantage que, dès lors que ces derniers ne sont plus en capacité de réagir avec le CO₂ pour le piéger, il suffit simplement de les remplacer par de nouveaux granulés de chaux sodée pour que le contenant 1 selon l'invention redevienne opérationnel.

La quantité de granulés de chaux sodée C est ajustée en fonction de la quantité de CO₂ à piéger et du volume disponible dans le compartiment supérieur 3 du contenant 1.

Selon un mode de réalisation, et comme visible plus particulièrement sur les figures 3 à 5, les granulés C de chaux sodée sont disposés directement sur le tamis 41, les ouvertures 42 du tamis 41 étant configurées de sorte à empêcher le passage des granulés C de chaux sodé du compartiment supérieur 3 au compartiment inférieur 2.

Cela permet avantageusement de simplifier la réalisation du contenant 1 selon un aspect de invention, en ce qu'il n'est pas nécessaire de prévoir un moyen supplémentaire pour assurer le maintien des granulés C de chaux sodée dans le compartiment supérieur 3.

Selon un mode de réalisation, et comme visible plus particulièrement sur les figures 3 à 5, 7A et 9B, le contenant comporte une ouverture supérieure 21 et le compartiment supérieur 3 comporte une ouverture inférieure 32.

L'ouverture supérieure 21 du contenant peut avantageusement être alignée avec l'ouverture inférieure 32 du compartiment supérieur 3.

Avantageusement, le moyen de séparation 4, et notamment le tamis 41, peut également être aligné avec l'ouverture supérieure 21 du contenant 1 et avec l'ouverture inférieure 32 du compartiment supérieur 3.

Ainsi, le contenant 1 peut présenter un encombrement réduit et assurer un passage optimal du CO₂ depuis le compartiment inférieur 2 vers le compartiment supérieur 3 à travers le moyen de séparation 4.

Le compartiment supérieur 3 est positionné intégralement dans le contenant 1. Avantageusement, et comme visible plus particulièrement sur la figure 5, un bouchon 5, notamment amovible, peut être positionné dans l'ouverture supérieure 21 du contenant, de sorte à rendre étanche ledit compartiment inférieur 2.

Grâce à cette disposition avantageuse de l'invention, l'étanchéité du contenant est assurée, de sorte que l'intégralité du CO₂ expulsé par l'échantillon de matière M au cours du temps passe nécessairement par le compartiment supérieur 3, et donc par les moyens C pour piéger le CO₂.

Alternativement, et sans sortir du cadre de la présente invention, l'étanchéité du compartiment inférieur 2 pourrait être assurée par le compartiment supérieur 3. Notamment, le compartiment supérieur 3 peut être configuré de sorte à obturer l'ouverture supérieure du compartiment inférieur 2, lorsque reçu, au moins en partie dans celui-ci. Également, un moyen d'étanchéité solidaire du compartiment supérieur 3 peut être prévu, et notamment un joint d'étanchéité positionné sur le pourtour de la paroi périphérique 35 du compartiment supérieur 3.

Avantageusement, le bouchon 5 peut être réalisé en matériau élastomère.

Selon un mode de réalisation, le bouchon 5 comporte un perçage traversant 51 positionné dans le prolongement de l'ouverture d'échappement 31 du compartiment supérieur 3, de sorte à permettre au gaz du compartiment supérieur 3 de s'en échapper via ladite ouverture d'échappement 31.

Selon un mode de réalisation, le moyen de séparation 4, et notamment le tamis 41, est disposé au niveau de l'ouverture inférieure 32 du compartiment supérieur 3.

Cela permet avantageusement de positionner les moyens C pour piéger le CO₂ au plus proche du compartiment inférieur 2, et donc de l'échantillon de matière M.

Selon un mode de réalisation, le moyen de séparation 4, et notamment le tamis 41, est prévu pour être solidarisé, et notamment de manière amovible, au compartiment supérieur 3, éventuellement par l'intermédiaire de moyens de solidarisation.

Notamment, lorsque ledit moyen de séparation 4, et notamment le tamis 41, est solidarisé de manière amovible au compartiment supérieur 3, cela facilite son démontage, par exemple pour effectuer sa maintenance.

Selon un mode de réalisation, et comme visible plus particulièrement sur les figures 4 et 9B, le compartiment supérieur 3 comporte une paroi inférieure 33 dans laquelle est ménagée l'ouverture inférieure 32.

Avantageusement, le tamis 41 peut présenter une largeur W41 supérieure à la largeur W32 de l'ouverture inférieure 32, de sorte à reposer en appui sur la paroi inférieure 33 du compartiment supérieur 3.

Ainsi, le tamis 41 peut avantageusement être maintenu en position dans le compartiment supérieur 3 en étant pris en « sandwich » entre ladite paroi inférieure 33 et les granulés C de chaux sodée.

Également, afin de renforcer le maintien en position du tamis 41 dans le compartiment supérieur, il peut également être prévu une bague élastique amovible, de largeur sensiblement égale à la largeur W41 du tamis 41, prévue pour appuyer sur le tamis 41 en demeurant immobile par rapport à la paroi inférieure 33 du compartiment supérieur 3, de sorte à le maintenir en appui contre la paroi inférieure 33 du compartiment supérieur 3 avec le tamis 41 intercalé entre la bague élastique et la paroi inférieure 33.

Selon un mode de réalisation, le compartiment supérieur 3 comporte un couvercle 34 amovible, prévu pour permettre l'accès à l'intérieur du compartiment supérieur 3 lorsqu'ôté.

Ledit couvercle 34 peut notamment former la partie supérieure 3I du compartiment supérieur 3, tel que décrit ci-dessus.

Le couvercle 34 amovible facilite ainsi l'accès à l'intérieur du compartiment supérieur 3, et notamment aux moyens C pour piéger le CO₂, notamment pour en effectuer la maintenance, par exemple pour changer les granulés C de chaux sodée.

Selon un mode de réalisation, le compartiment inférieur 2 et le compartiment supérieur 3 sont prévus pour être fixés de manière amovible.

Cette disposition avantageuse de l'invention facilite la maintenance du contenant 1 selon un aspect de invention, et permet de faciliter l'accès à l'intérieur du compartiment inférieur 2, et notamment pour pouvoir y positionner ou retirer l'échantillon de matière M. Cela permet de simplifier la conception du contenant 1 selon un aspect de l'invention en ce qu'il n'est pas nécessaire de prévoir une ouverture d'accès supplémentaire à l'intérieur du compartiment inférieur 2.

Selon un mode de réalisation, le contenant 1 comporte une paroi périphérique 22 de forme sensiblement cylindrique, et le compartiment supérieur 3 comporte également une paroi périphérique 35 de forme sensiblement cylindrique, d'axe confondu avec l'axe de la paroi périphérique 22 du compartiment inférieur 2.

Le bouchon 5 peut avantageusement présenter sensiblement une forme tronconique, de sorte à assurer l'étanchéité du contenant en épousant la forme de la paroi périphérique 22 du contenant.

Selon un mode de réalisation, le contenant 1 comprend en outre un moyen de raccordement 6, notamment amovible, configuré pour permettre le raccordement du compartiment supérieur 3 à la conduite souple, ledit moyen de raccordement 6 étant positionné au niveau de l'ouverture d'échappement 31 du compartiment supérieur 3, en s'étendant au travers de ladite ouverture d'échappement 31.

Comme visible sur les figures 4, 5, 8 et 9A, le moyen de raccordement 6 peut par exemple présenter une forme sensiblement tronconique, de sorte à faciliter le raccordement et le maintien de la conduite souple. Une ou plusieurs nervures annulaires peuvent être prévues sur le pourtour du moyen de raccordement 6, de sorte à faciliter le maintien en position de la conduite souple, sur celui-ci.

Le moyen de raccordement 6 peut ainsi présenter un perçage traversant 61 s'étendant entre une extrémité longitudinale inférieure 62 et une extrémité longitudinale supérieure 63. L'extrémité longitudinale inférieure 62 peut avantageusement être positionnée à l'intérieur du compartiment supérieur 3, tandis que l'extrémité longitudinale supérieure 63 peut être positionnée à l'extérieur du compartiment supérieur 3.

Un moyen de filtration 7, comme par exemple un film poreux, notamment en nylon, peut être fixé au niveau de l'extrémité longitudinale inférieure 62, de sorte à filtrer les gaz passant au travers dudit perçage traversant 61 et ainsi éviter le passage de poussières au travers dudit perçage traversant 61, qui risqueraient par exemple d'endommager un moyen de mesure de pression, positionné en aval.

Un tel moyen de connexion 6 présente une conception simple et un coût de revient réduit. Il peut notamment s'agir d'un moyen de connexion standard du commerce.

Selon un mode de réalisation, le moyen de raccordement 6 est maintenu en position par rapport au compartiment supérieur 3 par ladite ouverture d'échappement 31 du compartiment supérieur 3, ledit moyen de raccordement 6 s'étendant au travers du perçage traversant 51 du bouchon 5, notamment avec son perçage traversant 61 dans le prolongement du perçage traversant 51 du bouchon 5, et étant maintenu en position par rapport au bouchon 5 par ledit perçage traversant 51.

Grâce à cette disposition avantageuse de l'invention, le moyen de raccordement 6 peut également remplir une fonction de maintien en position du compartiment supérieur 3 par rapport au compartiment inférieur 2, en assurant une transmission d'effort entre le compartiment supérieur 3 et le bouchon 5, lequel est maintenu en position par rapport au compartiment inférieur 2.

Alternativement, ou en complément, afin d'assurer le maintien en position du compartiment supérieur 3 par rapport au contenant, le diamètre D35 de la paroi périphérique 35 du compartiment supérieur 3 peut être sensiblement égal au diamètre D22 de la paroi périphérique 22 du contenant, au jeu d'emboîtement près, et de sorte que la paroi périphérique 22 se trouve, au moins partiellement, en frottement contre la paroi périphérique 35 du compartiment supérieur 3.

Selon un mode de réalisation, le compartiment supérieur 3 comporte une paroi supérieure 36, l'ouverture d'échappement 31 étant ménagée dans ladite paroi supérieure 36.

Avantageusement, la paroi supérieure 36 peut être formée dans la partie supérieure 3S du compartiment supérieur 3 et/ou dans le couvercle 34.

L'invention concerne, comme visible sur la figure 10, un appareil 10 pour la détermination au cours du temps de la quantité de CO₂ absorbée par un échantillon de matière M organique contenant un agent levant, comme par exemple de la levure et/ou du levain et/ou de la poudre levante, en particulier de pâte boulangère, comprenant :
- un moyen de mesure de la pression 11,
- au moins un contenant 1 selon l'un des modes de réalisation d'un aspect de l'invention décrit ci-dessus.

Le moyen de mesure de la pression 11 est relié à l'ouverture d'échappement 31 du compartiment supérieur 3 du contenant 1 de sorte à pouvoir déterminer l'évolution au cours du temps de la pression dans le compartiment inférieur 2 recevant l'échantillon de matière M, après passage des gaz issus du compartiment inférieur 2 dans le compartiment supérieur 3 du contenant 1.

Un tel appareil 10 permet avantageusement de connaître de manière simple et fiable l'évolution de la pression dans le compartiment inférieur 2 du contenant 1 due à la capacité de rétention du CO₂ par ledit échantillon de matière M, et notamment afin de connaître l'évolution au cours du temps de la quantité de CO₂ retenue au cours du temps par un échantillon de matière M, et notamment de matière organique contenant un agent levant comme par exemple de la levure et/ou du levain et/ou de la poudre levante, en particulier de la pâte boulangère.

Avantageusement, l'appareil peut être réalisé à partir d'un appareil du commerce, comme par exemple l'appareil commercialisé sous le nom de RISOGRAPH ^{®}, décrit ci-dessus, avec seulement quelques adaptations.

L'appareil 10 peut avantageusement comporter plusieurs contenants 1 selon un aspect de l'invention, de sorte à effectuer des mesures de pression simultanées des différents contenants 1, et de sorte à déterminer simultanément l'évolution de la pression au cours du temps dans les compartiments inférieurs 2 de ces contenants 1, et notamment afin de connaître l'évolution au cours du temps de la quantité de CO₂ retenue au cours du temps par une pluralité d'échantillons de matière M.

Le moyen de mesure de la pression 11 est connecté à l'ouverture d'échappement 31 du compartiment supérieur 3 du contenant 1, notamment via le bouchon 5 et/ou le moyen de raccordement 6, par l'intermédiaire d'une conduite P souple.

Le moyen de mesure de la pression 11 peut par exemple être un capteur de pression relié à une unité de commande.

L'ensemble des dispositions décrites précédemment concernant un appareil pour la détermination au cours du temps de la quantité de CO₂ absorbée par un échantillon de matière M s'appliquent à l'appareil 10 selon l'invention.

L'invention concerne encore un procédé de détermination au cours du temps de la quantité de CO₂ absorbée par un échantillon de matière M (à savoir retenu par l'échantillon), et notamment de matière organique contenant un agent levant, comme par exemple de la levure et/ou du levain et/ou de la poudre levante, en particulier de pâte boulangère, comprenant :
/a/ fourniture d'un appareil selon l'un des modes de réalisation de l'invention décrits ci-dessus ;
/b/ mise en place de l'échantillon de matière M dans le compartiment inférieur 2 du contenant 1,
/c/ mesure de l'évolution de la pression au cours du temps dans le compartiment inférieur 2 recevant l'échantillon de matière M, après passage des gaz issus du compartiment inférieur 2 dans le compartiment supérieur 3 du contenant 1.

Le procédé selon l'invention est mis en œuvre dans l'appareil 10 selon un mode de réalisation de l'invention, tel que décrit ci-dessus.

Lors de la mise en œuvre un tel procédé, la variation de pression est due à la fermentation génératrice de CO₂, qui est retenu par l'échantillon (à savoir le CO₂ absorbé), et non au CO₂ expulsé par l'échantillon puisque le CO₂ expulsé est éliminé par les moyens pour piéger le CO₂.

La réaction de fermentation ne générant que du gaz CO₂, on peut déterminer par calcul une quantité de CO₂ absorbé (retenu par l'échantillon) par la connaissance du volume interne du contenant, auquel on additionne, le volume interne de la conduite souple reliant l'ouverture d'échappement au moyen de mesure de pression.

Un tel procédé permet avantageusement de connaître de manière simple et fiable l'évolution de la pression dans le compartiment inférieur 3 du contenant 1 engendrée par rétention de CO₂ par l'échantillon de matière M, et notamment afin de connaître l'évolution au cours du temps de la quantité de CO₂ retenue au cours du temps par un échantillon de matière M, et notamment de matière organique contenant un agent levant, comme par exemple de la levure et/ou du levain et/ou de la poudre levante, en particulier de la pâte boulangère, au cours de la réaction de fermentation..

La présente divulgation est encore relative à un procédé de détermination au cours du temps de la quantité de CO₂ expulsé par un échantillon de matière organique contenant de la levure et/ou du levain et/ou de la poudre levante, en particulier de pâte boulangère au cours d'une réaction de fermentation génératrice de CO₂.

Un tel procédé comprend simultanément, une première mesure sur une première fraction d'échantillon et une deuxième mesure et une deuxième fraction d'échantillon. La première fraction et la deuxième fraction ont un même volume et sont typiquement obtenues sur un échantillon, en particulier une pâte boulangère fraîchement préparée.

La première mesure est configurée pour mesurer l'évolution de pression due à la quantité de gaz absorbé uniquement et donc permet la détermination de la quantité du CO₂ absorbé (ou retenu), comme précédemment expliqué, alors que la deuxième mesure est configurée pour mesurer l'évolution de pression due non seulement à la quantité de gaz absorbé, mais encore à la quantité de gaz expulsé et donc permet la détermination de la quantité du CO₂ totale, générée par la fermentation (absorbée et expulsée)

On détermine la quantité du CO₂ expulsé par différence entre la deuxième mesure et la première mesure.

En particulier la première mesure comprend :
/a1/ fourniture d'un premier appareil selon la présente divulgation comprenant les moyens C pour piéger le CO2 reçus dans le compartiment supérieur du contenant ;
/b1/ mise en place de la première fraction de échantillon de matière dans le compartiment inférieur 2 du contenant 1,
/c1/ mesure de l'évolution de la pression au cours du temps dans le compartiment inférieur 2 recevant l'échantillon de matière M, après passage des gaz issus du compartiment inférieure 2 dans le compartiment supérieur 3 du contenant 1, le CO2 expulsé par l'échantillon éliminé par les moyens pour piéger le CO2.

La deuxième mesure, réalisée simultanément à la première mesure comprend :
/a2/ fourniture d'un deuxième appareil selon la présente divulgation, mais dépourvu des moyens C pour piéger le CO₂ reçus dans le compartiment supérieur 3 du contenant 1;
/b2/ mise en place de la deuxième fraction de l'échantillon de matière M dans le compartiment inférieur 2 du contenant 1,
/c2/ mesure de l'évolution de la pression au cours du temps dans le compartiment inférieur 2 recevant la deuxième fraction de échantillon de matière M, après passage des gaz issus du compartiment inférieure 2 dans le compartiment supérieur 3 du contenant 1, dépourvu des moyens pour piéger le CO₂.

Un tel procédé permet avantageusement de connaître l'évolution au cours du temps de la quantité de CO₂ expulsée au cours du temps par un échantillon de matière M, et notamment de matière organique contenant un agent levant, comme par exemple de la levure et/ou du levain et/ou de la poudre levante, en particulier de la pâte boulangère, au cours de la réaction de fermentation.

Comme visible sur les courbes des graphiques des figures 2A et 2B, le procédé selon l'invention permet par exemple de connaître l'évolution au cours du temps de la vitesse d'expulsion/rétention de CO₂ par un échantillon de matière M, et notamment d'une pâte boulangère. La courbe en dents de scie de la figure 2A montre en effet l'évolution de la vitesse d'expulsion de CO₂ d'un échantillon de pâte boulangère.

Également, comme expliqué ci-dessus, le procédé selon l'invention permet également de déterminer de manière particulièrement fiable, l'influence de différents paramètres sur l'évolution au cours du temps de la quantité de CO₂ retenue/expulsée par un échantillon de matière M, et notamment de matière organique contenant un agent levant, comme par exemple de la levure et/ou du levain et/ou de la poudre levante, en particulier de pâte boulangère. Notamment, comme expliqué ci-dessus et comme visible sur le graphique de la figure 2D, les inventeurs ont pu déterminer que l'ajout d'un ingrédient A dans une composition de pâte boulangère, n'avait pas d'influence sur la production totale de CO₂ par la pâte boulangère mais avait une influence sur la quantité de CO₂ retenue/expulsée par ladite pâte boulangère au cours du temps, ce qui n'aurait pas été possible avec les procédés de l'art antérieur.

L'ensemble des dispositions décrites précédemment concernant la détermination au cours du temps de la quantité de CO₂ absorbée et/ou expulsée par un échantillon de matière M reçu dans le compartiment inférieur 2 d'un contenant 1 selon l'un des modes de réalisation de l'invention décrit ci-dessus s'appliquent au procédé selon l'invention.

Naturellement, d'autres modes de réalisation auraient pu être envisagés par l'Homme du métier sans pour autant sortir du cadre de l'invention définie par les revendications ci-après.

### Liste des signes de référence

1. Contenant
2. Compartiment inférieur
21. Ouverture supérieure
22. Paroi périphérique
D22. Diamètre
3. Compartiment supérieur
31. Partie inférieure
3S. Partie supérieure
31. Ouverture d'échappement
32. Ouverture inférieure
33. Paroi inférieure
34. Couvercle
35. Paroi périphérique
D35. Diamètre
36. Paroi supérieure
W32. Largeur
4. Moyen de séparation
41. Tamis
W41. Largeur
42. Ouverture
5. Bouchon
51. Perçage traversant
6. Moyen de raccordement
61. Perçage traversant
62. Extrémité longitudinale inférieure
63. Extrémité longitudinale supérieure
7. Moyen de filtration
10. Appareil
11. Moyen de mesure de la pression
C. Moyens pour piéger le CO₂/Granulés de chaux sodée
M. Echantillon de matière
P. Conduite

## Revendications

1. Appareil (10) pour la détermination de la quantité de CO₂ absorbée par un échantillon de matière (M) organique contenant de la levure et/ou du levain et/ou de la poudre levante, en particulier de pâte boulangère, au cours du temps, comprenant :
- un moyen de mesure de pression (11), et
- au moins un contenant (1) comprenant :
- - un compartiment inférieur (2) prévu pour recevoir ledit échantillon de matière (M),
- -un compartiment supérieur (3) recevant des moyens (C) pour piéger le CO₂, positionné dans le prolongement et communiquant avec le compartiment inférieur (2), et présentant une ouverture d'échappement (31) permettant au gaz de s'échapper du compartiment supérieur (3) après son passage par lesdits moyens (C) pour piéger le CO₂,
- - un moyen de séparation (4), disposé entre le compartiment inférieur (2) et le compartiment supérieur (3), configuré pour autoriser le passage de gaz depuis le compartiment inférieur (2) vers le compartiment supérieur (3),
dans lequel le moyen de mesure de la pression (11) est relié à l'ouverture d'échappement (31) du compartiment supérieur (3) du contenant (1) par une conduite souple (P) de sorte à pouvoir déterminer l'évolution au cours du temps de la pression dans le compartiment inférieur (2) recevant l'échantillon de matière (M) ,après passage des gaz issus du compartiment inférieur (2) dans le compartiment supérieur (3) du contenant (1).

2. Appareil selon la revendication 1 dans lequel le compartiment inférieur (2) est au moins partiellement, de préférence intégralement, dans un matériau transparent, par exemple en verre ou encore en matériau thermoplastique, de sorte à permettre à un observateur de visualiser le contenu du compartiment inférieur (2).

3. Appareil (10) selon la revendication 1 ou 2, dans lequel le compartiment inférieur (2), le compartiment supérieur (3) et le moyen de séparation (4) forment un ensemble autoportant.

4. Appareil (10) selon l'une des revendications 1 à 3, dans lequel le moyen de séparation (4) comporte un tamis (41) dans lequel sont ménagées une pluralité d'ouvertures (42) configurées de sorte à autoriser le passage de gaz depuis le compartiment inférieur (2) vers le compartiment supérieur (3).

5. Appareil (10) selon l'une des revendications 1 à 4, dans lequel les moyens (C) pour piéger le CO₂ comportent des granulés (C) de chaux sodée.

6. Appareil (10) selon les revendications 4 et 5, dans lequel les granulés (C) de chaux sodée sont disposés directement sur le tamis (41), les ouvertures (42) du tamis (41) étant configurées de sorte à empêcher le passage des granulés (C) de chaux sodée du compartiment supérieur (3) au compartiment inférieur (2).

7. Appareil (10) selon l'une des revendications 1 à 6, dans lequel le contenant (2) comporte une ouverture supérieure (21) et le compartiment supérieur (3) comporte une ouverture inférieure (32), l'ouverture supérieure (21) du contenant (1) étant alignée avec l'ouverture inférieure (32) du compartiment supérieur (3), et dans lequel le moyen de séparation (4) est également aligné avec l'ouverture supérieure (21) du contenant (1) et avec l'ouverture inférieure (32) du compartiment supérieur (3).

8. Appareil (10) selon la revendication 7, dans lequel un bouchon (5), amovible est positionné dans l'ouverture supérieure (21) du contenant (1), de sorte à rendre étanche ledit contenant (1).

9. Appareil (10) selon la revendication 8, dans lequel le bouchon (5) comporte un perçage traversant (51) positionné dans le prolongement de l'ouverture d'échappement (31) du compartiment supérieur (3), de sorte à permettre au gaz du compartiment supérieur (3) de s'en échapper via ladite ouverture d'échappement (31).

10. Appareil (10) selon l'une des revendications 7 à 9, dans lequel le moyen de séparation (4) est disposé au niveau de l'ouverture inférieure (32) du compartiment supérieur (3).

11. Appareil (10) selon l'une des revendications 1 à 10, dans lequel le compartiment inférieur (2) et le compartiment supérieur (3) sont fixés de manière amovible.

12. Appareil (10) selon l'une des revendications 1 à 11, dans lequel le contenant (1) comporte une paroi périphérique (22) de forme sensiblement cylindrique, et le compartiment supérieur (3) comporte également une paroi périphérique (35) de forme sensiblement cylindrique, d'axe confondu avec l'axe de la paroi périphérique (22) du contenant (1).

13. Appareil (10) selon l'une des revendications 1 à 12, comprenant en outre un moyen de raccordement (6), notamment amovible, configuré pour permettre le raccordement du compartiment supérieur (3) à la conduite souple, ledit moyen de raccordement (6) étant positionné au niveau de l'ouverture d'échappement (31) du compartiment supérieur (3), en s'étendant au travers de ladite ouverture d'échappement (31).

14. Appareil (10) selon la revendication 13, prise en combinaison avec la revendication 9, dans lequel le moyen de raccordement (6) est maintenu en position par rapport au compartiment supérieur (3) par ladite ouverture d'échappement (31) du compartiment supérieur (3), ledit moyen de raccordement (6) s'étendant au travers du perçage traversant (51) du bouchon (5) et étant maintenu en position par rapport au bouchon (5) par ledit perçage traversant (51).

15. Appareil (10) selon l'une des revendications 1 à 14, dans lequel le compartiment supérieur (3) comporte une paroi supérieure (36), l'ouverture d'échappement (31) étant ménagée dans ladite paroi supérieure (36).

16. Procédé de détermination au cours du temps de la quantité de CO2 absorbée par un échantillon de matière organique contenant de la levure et/ou du levain et/ou de la poudre levante, en particulier de pâte boulangère au cours d'une réaction de fermentation génératrice de CO2, comprenant :
/a/ fourniture d'un appareil selon l'une des revendications 1 à 15
/b/ mise en place de l'échantillon de matière (M) dans le compartiment inférieur (2) du contenant (1),
/c/ mesure de l'évolution de la pression au cours du temps dans le compartiment inférieur (2) recevant l'échantillon de matière (M), après passage des gaz issus du compartiment inférieure (2) dans le compartiment supérieur (3) du contenant (1), le CO₂ expulsé par l'échantillon éliminé par les moyens pour piéger le CO2.

17. Procédé de détermination au cours du temps de la quantité de CO₂ expulsé par un échantillon de matière organique contenant de la levure et/ou du levain et/ou de la poudre levante, en particulier de pâte boulangère au cours d'une réaction de fermentation génératrice de CO₂, comprenant simultanément, une première mesure et une deuxième mesure sur une première fraction d'échantillon et une deuxième fraction d'échantillon, de même volume, et dans lequel ladite première mesure est configurée pour mesurer l'évolution de pression due à la quantité de gaz absorbé uniquement et la deuxième mesure est configurée pour mesurer l'évolution de pression due à la quantité de gaz absorbé et à la quantité de gaz expulsé,
et dans la première mesure comprend :
/a1/ fourniture d'un premier appareil selon l'une des revendications 1 à 15
/b1/ mise en place de la première fraction de échantillon de matière (M) dans le compartiment inférieur (2) du contenant (1),
/c1/ mesure de l'évolution de la pression au cours du temps dans le compartiment inférieur (2) recevant l'échantillon de matière (M), après passage des gaz issus du compartiment inférieure (2) dans le compartiment supérieur (3) du contenant (1), le CO₂ expulsé par l'échantillon éliminé par les moyens pour piéger le CO₂
et dans lequel la deuxième mesure comprend :
/a2/ fourniture d'un deuxième appareil selon l'une des revendications 1 à 15 dépourvu des moyens (C) pour piéger le CO₂ reçus dans le compartiment supérieur (3) du contenant (1),
/b2/ mise en place de la deuxième fraction de échantillon de matière (M) dans le compartiment inférieur (2) du contenant (1),
/c2/ mesure de l'évolution de la pression au cours du temps dans le compartiment inférieur (2) recevant la deuxième fraction de échantillon de matière (M), après passage des gaz issus du compartiment inférieure (2) dans le compartiment supérieur (3) du contenant (1), dépourvu des moyens pour piéger le CO₂
et dans lequel on obtient la quantité de CO₂ expulsé par l'échantillon à partir de la différence entre la deuxième mesure et la première mesure.

## Patentansprüche

1. Vorrichtung (10) zur Bestimmung der Menge an CO₂, die von einer Probe organischen Materials (M), das Hefe und/oder Sauerteig und/oder Backpulver enthält, insbesondere von Backteig, im Laufe der Zeit absorbiert wird, umfassend:
- eine Druckmessvorrichtung (11), und
- mindestens einen Behälter (1), umfassend:
- - ein unteres Fach (2) zur Aufnahme der Materialprobe (M),
- - ein oberes Fach (3) zur Aufnahme von Mitteln (C) zum Auffangen von CO₂, das in Verlängerung des unteren Fachs (2) angeordnet ist und mit diesem in Verbindung steht, und eine Auslassöffnung (31) aufweist, durch die das Gas aus dem oberen Fach (3) entweichen kann, nachdem es die Mittel (C) zum Auffangen von CO₂ passiert hat,
- - ein Trennmittel (4), das zwischen dem unteren Fach (2) und dem oberen Fach (3) angeordnet ist und dazu ausgebildet ist, den Durchgang von Gas aus dem unteren Fach (2) in das obere Fach (3) zu ermöglichen,
wobei die Druckmessvorrichtung (11) mit der Auslassöffnung (31) des oberen Fachs (3) des Behälters (1) über eine flexible Leitung (P) verbunden ist, um die zeitliche Entwicklung des Drucks im unteren Fach (2), das die Materialprobe (M) aufnimmt, nach dem Durchgang der Gase aus dem unteren Fach (2) in das obere Fach (3) des Behälters (1) bestimmen zu können.

2. Vorrichtung nach Anspruch 1, wobei das untere Fach (2) wenigstens teilweise, bevorzugt vollständig, aus einem transparenten Material besteht, beispielsweise aus Glas oder auch aus einem thermoplastischen Material, das einem Beobachter ermöglicht, den Inhalt des unteren Raums (2) zu sehen.

3. Vorrichtung (10) nach Anspruch 1 oder 2, wobei das untere Fach (2), das obere Fach (3) und das Trennmittel (4) eine selbsttragende Einheit bilden.

4. Vorrichtung (10) nach einem der Ansprüche 1 bis 3, wobei das Trennmittel (4) ein Sieb (41) umfasst, in dem eine Mehrzahl von Öffnungen (42) ausgebildet ist, die dazu ausgebildet sind, den Durchgang von Gas aus dem unteren Fach (2) in das obere Fach (3) zu ermöglichen.

5. Vorrichtung (10) nach einem der Ansprüche 1 bis 4, wobei die Mittel (C) zum Auffangen von CO₂ Granulat (C) aus Natriumkalk umfassen.

6. Vorrichtung (10) nach den Ansprüchen 4 und 5, wobei das Granulat (C) aus Natriumkalk direkt auf dem Sieb (41) angeordnet ist, wobei die Öffnungen (42) des Siebs (41) so ausgebildet sind, dass sie den Durchgang des Granulats (C) aus Natriumkalk vom oberen Fach (3) zum unteren Fach (2) verhindern.

7. Vorrichtung (10) nach einem der Ansprüche 1 bis 6, wobei der Behälter (2) eine obere Öffnung (21) aufweist und das obere Fach (3) eine untere Öffnung (32) aufweist, wobei die obere Öffnung (21) des Behälters (1) mit der unteren Öffnung (32) des oberen Fachs (3) ausgerichtet ist und wobei das Trennmittel (4) ebenfalls mit der oberen Öffnung (21) des Behälters (1) und mit der unteren Öffnung (32) des oberen Fachs (3) ausgerichtet ist.

8. Vorrichtung (10) nach Anspruch 7, wobei ein abnehmbarer Verschluss (5) in der oberen Öffnung (21) des Behälters (1) positioniert ist, um den Behälter (1) abzudichten.

9. Vorrichtung (10) nach Anspruch 8, wobei der Verschluss (5) eine Durchgangsbohrung (51) aufweist, die in Verlängerung der Auslassöffnung (31) des oberen Fachs (3) angeordnet ist, so dass das Gas aus dem oberen Fach (3) über die Auslassöffnung (31) entweichen kann.

10. Vorrichtung (10) nach einem der Ansprüche 7 bis 9, wobei das Trennmittel (4) an der unteren Öffnung (32) des oberen Fachs (3) angeordnet ist.

11. Vorrichtung (10) nach einem der Ansprüche 1 bis 10, wobei der untere Behälter (2) und der obere Behälter (3) lösbar befestigt sind.

12. Vorrichtung (10) nach einem der Ansprüche 1 bis 11, wobei der Behälter (1) eine im Wesentlichen zylindrische Umfangswand (22) aufweist und das obere Fach (3) ebenfalls eine im Wesentlichen zylindrische Umfangswand (35) aufweist, deren Achse mit der Achse der Umfangswand (22) des Behälters (1) zusammenfällt.

13. Vorrichtung (10) nach einem der Ansprüche 1 bis 12, welche ferner ein insbesondere abnehmbares Verbindungsmittel (6) umfasst, das dazu ausgebildet ist, die Verbindung des oberen Fachs (3) mit der flexiblen Leitung zu ermöglichen, wobei das Verbindungsmittel (6) an der Auslassöffnung (31) des oberen Fachs (3) positioniert ist und sich durch die Auslassöffnung (31) erstreckt.

14. Vorrichtung (10) nach Anspruch 13 in Kombination mit Anspruch 9, wobei die Verbindungsmittel (6) durch die Auslassöffnung (31) des oberen Fachs (3) in ihrer Position relativ zum oberen Fach (3) gehalten wird, wobei sich das Verbindungsmittel (6) durch die Durchgangsbohrung (51) des Stopfens (5) erstreckt und durch die Durchgangsbohrung (51) in seiner Position relativ zum Stopfen (5) gehalten wird.

15. Vorrichtung (10) nach einem der Ansprüche 1 bis 14, wobei das obere Fach (3) eine obere Wand (36) aufweist, wobei die Auslassöffnung (31) in der oberen Wand (36) ausgenommen ist.

16. Verfahren zur zeitlichen Bestimmung der von einer Probe organischen Materials, das Hefe und/oder Sauerteig und/oder Backpulver enthält, insbesondere von Backteig, während einer CO₂-erzeugenden Fermentationsreaktion absorbierten CO₂-Menge, umfassend:
/a/ Bereitstellen einer Vorrichtung nach einem der Ansprüche 1 bis 15,
/b/ Einbringen der Materialprobe (M) in das untere Fach (2) des Behälters (1),
/c/ Messung der Druckentwicklung im Laufe der Zeit im unteren Fach (2), das die Materialprobe (M) enthält, nachdem die aus dem unteren Fach (2) stammenden Gase in das obere Fach (3) des Behälters (1) gelangt sind, wobei das von der Probe ausgestoßene CO₂ durch die Mittel zum Auffangen von CO₂ entfernt wird.

17. Verfahren zur zeitlichen Bestimmung der von einer Probe organischen Materials, das Hefe und/oder Sauerteig und/oder Backpulver enthält, insbesondere von Backteig, während einer CO₂-erzeugenden Fermentationsreaktion absorbierten CO₂-Menge, umfassend gleichzeitig eine erste Messung und eine zweite Messung an einer ersten Probenfraktion und einer zweiten Probenfraktion mit gleichem Volumen, wobei die erste Messung dazu ausgebildet ist, die Druckänderung aufgrund der Menge des absorbierten Gases allein zu messen, und die zweite Messung dazu ausgebildet ist, die Druckänderung aufgrund der Menge des absorbierten Gases und der Menge des ausgestoßenen Gases zu messen,
und wobei die erste Messung umfasst:
/a1/ Bereitstellung einer ersten Vorrichtung nach einem der Ansprüche 1 bis 15,
/b1/ Einbringen der ersten Materialprobenfraktion (M) in den unteren Raum (2) des Behälters (1),
/c1/ Messung der Druckänderung im Laufe der Zeit in dem unteren Fach (2), das die Materialprobe (M) aufnimmt, nachdem die aus dem unteren Fach (2) stammenden Gase in das obere Fach (3) des Behälters (1) gelangt sind, wobei das aus der Probe ausgestoßene CO₂ durch die Mittel zum Auffangen von CO₂ entfernt wird und wobei die zweite Messung umfasst:
/a2/ Bereitstellung einer zweiten Vorrichtung nach einem der Ansprüche 1 bis 15, die keine Mittel (C) zum Auffangen von CO₂ aufweist, das in dem oberen Fach (3) des Behälters (1) aufgenommen wird,
/b2/ Einbringen der zweiten Materialprobenfraktion (M) in das untere Fach (2) des Behälters (1),
/c2/ Messen der Druckänderung im Laufe der Zeit in dem unteren Fach (2), das die zweite Materialprobenfraktion (M) aufnimmt, nachdem die Gase aus dem unteren Fach (2) in das obere Fach (3) des Behälters (1) gelangt sind, das keine Mittel zum Auffangen des CO₂ aufweist und in dem die von der Probe ausgestoßene CO₂-Menge aus der Differenz zwischen der zweiten Messung und der ersten Messung ermittelt wird.

## Claims

1. Apparatus (10) for determining the amount of CO₂ absorbed by a sample of organic matter (M) containing yeast and/or leaven and/or baking powder, particularly baking dough, over time, comprising:
- a pressure measuring means (11), and
- at least one container (1) comprising:
- - a lower compartment (2) designed to hold said sample of matter (M),
- - an upper compartment (3) holding means (C) for trapping CO₂, positioned in line with and communicating with the lower compartment (2), and featuring an exhaust opening (31) that allows the gas to escape from the upper compartment (3) after passing through said means (C) for trapping CO₂,
- - a separating means (4), arranged between the lower compartment (2) and the upper compartment (3), designed to permit the flow of gas from the lower compartment (2) to the upper compartment (3),
wherein the pressure measuring means (11) is connected to the exhaust opening (31) of the upper compartment (3) of the container (1) via a flexible pipe (P), so as to be able to determine the pressure changes over time in the lower compartment (2) receiving the sample of matter (M), after the gases from the lower compartment (2) have passed into the upper compartment (3) of the container (1).

2. Apparatus according to Claim 1, wherein the lower compartment (2) is at least partially, preferably entirely, made of a transparent material, such as glass or thermoplastic, allowing an observer to see the contents of the lower compartment (2).

3. Apparatus (10) according to Claim 1 or 2, wherein the lower compartment (2), the upper compartment (3) and the separating means (4) form a self-supporting assembly.

4. Apparatus (10) according to one of Claims 1 to 3, wherein the separating means (4) includes a screen (41) in which a plurality of openings (42) are arranged designed to allow the passage of gas from the lower compartment (2) to the upper compartment (3).

5. Apparatus (10) according to one of Claims 1 to 4, wherein the means (C) for trapping the CO₂ include soda lime granules (C).

6. Apparatus (10) according to Claims 4 and 5, wherein the soda lime granules (C) are arranged directly on the screen (41), the openings (42) of the screen (41) being designed so as to prevent the passage of the soda lime granules (C) from the upper compartment (3) to the lower compartment (2).

7. Apparatus (10) according to any one of Claims 1 to 6, wherein the container (2) includes an upper opening (21) and the upper compartment (3) includes a lower opening (32), the upper opening (21) of the container (1) being aligned with the lower opening (32) of the upper compartment (3), and wherein the separating means (4) is also aligned with the upper opening (21) of the container (1) and with the lower opening (32) of the upper compartment (3).

8. Apparatus (10) according to Claim 7, wherein a removable stopper (5) is positioned in the upper opening (21) of the container (1), so as to seal said container (1).

9. Apparatus (10) according to Claim 8, wherein the stopper (5) features a through-hole (51) aligned with the exhaust opening (31) of the upper compartment (3), so as to allow gas from the upper compartment (3) to escape through said exhaust opening (31).

10. Apparatus (10) according to one of Claims 7 to 9, wherein the separating means (4) is arranged at the lower opening (32) of the upper compartment (3).

11. Apparatus (10) according to any one of Claims 1 to 10, wherein the lower compartment (2) and the upper compartment (3) are removably secured.

12. Apparatus (10) according to one of Claims 1 to 11, wherein the container (1) has a peripheral wall (22) of substantially cylindrical shape, and the upper compartment (3) also has a peripheral wall (35) of substantially cylindrical shape, with its axis coinciding with the axis of the peripheral wall (22) of the container (1).

13. Apparatus (10) according to one of Claims 1 to 12, further comprising a connection means (6), in particular a removable one, designed to enable the connection of the upper compartment (3) to the flexible pipe, said connection means (6) being positioned at the exhaust opening (31) of the upper compartment (3), extending through said exhaust opening (31).

14. Apparatus (10) according to Claim 13, in combination with Claim 9, wherein the connection means (6) is secured in position relative to the upper compartment (3) by said exhaust opening (31) of the upper compartment (3), said connection means (6) extending through the through-hole (51) of the stopper (5) and being retained in position relative to the stopper (5) by said through-hole (51).

15. Apparatus (10) according to one of Claims 1 to 14, wherein the upper compartment (3) includes an upper wall (36), the exhaust opening (31) being provided in said upper wall (36).

16. Method for determining the amount of CO₂ absorbed by a sample of organic matter containing yeast and/or leaven and/or baking powder, particularly baking dough, over time, during a CO2-generating fermentation reaction, comprising:
/a/ providing an apparatus according to one of Claims 1 to 15
/b/ placing the sample of matter (M) in the lower compartment (2) of the container (1),
/c/ measuring changes in the pressure over time in the lower compartment (2) receiving the sample of matter (M), after the gases from the lower compartment (2) have passed into the upper compartment (3) of the container (1), the CO₂ expelled by the sample eliminated by the means for trapping CO2.

17. Method for determining the amount of CO₂ expelled by a sample of organic matter containing yeast and/or leaven and/or baking powder, particularly baking dough, over time, during a CO₂-generating fermentation reaction, comprising simultaneously a first measurement and a second measurement on a first sample fraction and a second sample fraction of the same volume, and wherein said first measurement is configured to measure the change in pressure due solely to the amount of absorbed gas, while the second measurement is configured to measure the change in pressure due to both the amount of absorbed gas and the amount of expelled gas,
and wherein the first measurement comprises:
/a1/ providing a first apparatus according to one of Claims 1 to 15
/b1/ placing the first sample fraction of matter (M) in the lower compartment (2) of the container (1),
/c1/ measuring changes in the pressure over time in the lower compartment (2) receiving the sample of matter (M), after the gases from the lower compartment (2) have passed into the upper compartment (3) of the container (1), the CO₂ expelled by the sample eliminated by the means for trapping CO₂
and wherein the second measurement comprises:
/a2/ providing a second apparatus according to one of Claims 1 to 15 devoid of means (C) for trapping CO₂ received in the upper compartment (3) of the container (1);
/b2/ placing the second sample fraction of matter (M) in the lower compartment (2) of the container (1),
/c2/ measuring changes in the pressure over time in the lower compartment (2) receiving the second sample fraction of matter (M), after the gases from the lower compartment (2) have passed into the upper compartment (3) of the container (1), devoid of means for trapping CO₂
and wherein the amount of CO₂ expelled by the sample is obtained from the difference between the second measurement and the first measurement.
